Europäisches Patentamt

European Patent Office

Office européen des brevets

(11) Publication number: **0 367 475**
**A2**

## EUROPEAN PATENT APPLICATION

(21) Application number: 89310931.4

(22) Date of filing: 24.10.89

(51) Int. Cl.5 **C07K 3/12 , C07K 15/22 ,
//A61K37/14**

(30) Priority: 29.10.88 JP 275664/88

(43) Date of publication of application:
09.05.90 Bulletin 90/19

(84) Designated Contracting States:
DE FR GB IT

(71) Applicant: NIPPON OIL AND FATS COMPANY,
LIMITED
10-1, Yuraku-cho 1-chome
Chiyoda-ku Tokyo 100(JP)

(72) Inventor: Tsuchida, Eishun
10-10 Seckimachiminami 2-chome Nerima-ku
Tokyo(JP)
Inventor: Ohno, Hiroyuki
104 Nakakasai Sukai Haitsu 11-25 Nakakasai
5-chome
Edogawa-ku Tokyo(JP)
Inventor: Takeoka, Shinji c/o Mr. Hisashi
Soma
18-20 Hyakunincho 3-chome
Shinjuku-ku Tokyo(JP)

(74) Representative: Ackroyd, Robert et al
Pollak, Mercer & Tench Eastcheap House
Central Approach
Letchworth Hertfordshire SG6 3DS(GB)

(54) Process for producing haemoglobin concentrate.

(57) A process for producing haemoglobin concentrate comprises concentrating erythrocytes as much as possible, destroying the membranes of the erythrocytes to obtain a haemoglobin-concentrated solution and removing the constituents of the erythrocyte membranes and the like without diluting the solution.

EP 0 367 475 A2

## PROCESS FOR PRODUCING HAEMOGLOBIN CONCENTRATE

The present invention relates to processes for producing haemoglobin concentrates.

Haemoglobins concentrated to about 30% by weight are contained naturally in erythrocytes. Conventional basic methods for producing haemoglobin concentrates employ a procedure for concentration of the haemoglobins: after haemolyzing erythrocytes and separating haemoglobins, the haemoglobins obtained are concentrated. As a method of haemolyzing the erythrocytes, a method in which a shock of osmotic pressure is given to the erythrocytes by adding a hypotonic solution is generally used. However, in this method, since the haemoglobins obtained are diluted to 8% by weight, concentration of the haemoglobins is required. Effective methods for the concentration of the haemoglobins have however not been available. Specifically, the precipitation method and the dispersion adsorption method which are commonly used for the concentration of proteins are not suitable for the concentration of haemoglobins because a solvent other than a physiological sodium chloride solution is used. In freezing and drying methods, haemoglobins are denatured. Accordingly, an ultrafiltration method has been used for the concentration of haemoglobins. However, in this method, as the haemoglobins are concentrated, the filter is often clogged and the operation becomes troublesome and inefficient. For these reasons, there is a demand for more efficient methods for the concentration of haemoglobins.

The present inventors have conducted research for resolving the above problems concerning methods of protein concentration and, especially, have made many basic studies of purification of haemoglobins. They then found a method for efficiently producing haemoglobin concentrate.

The present invention provides a process for producing haemoglobin concentrate which comprises concentrating erythrocytes, haemolyzing the erythrocyte concentrate, and removing impurities, for example membrane constituents (stroma), from the haemolyzed concentrate to obtain the separated and purified haemoglobin concentrate.

The following description illustrates the invention more specifically.

The method of the invention comprises three steps, namely the concentration of erythrocytes, the haemolyzation of the concentrate and the separation and purification of haemoglobins.

When the erythrocytes are concentrated, a centrifuging method may be used. The erythrocytes may be washed with a physiological sodium chloride solution and concentrated by using a centrifugal separator to separate the erythrocytes.

Since the erythrocytes have a diameter of about 8 μm, it is easy to concentrate them by using a centrifugal separator. Furthermore, after the erythrocytes have been washed with physiological sodium chloride solution, a hypertonic solution in which the erythrocytes do not haemolyze is preferably added to the washed erythrocytes to contact them; the contracted erythrocytes can then be efficiently concentrated and separated by centrifugal operation. Moreover, the washing of the erythrocytes can be also conducted by centrifugal operation.

The erythrocytes are next haemolyzed. As a haemolyzing method for separating the haemoglobins from the erythrocytes, a method in which the equivalent volume of a hypotonic solution is added is commonly used. As another haemolyzing method, an ultrasonic exposure method or an electric perforation method can be used. The most preferred haemolyzing method however is a freezing and thawing method. In one process embodying the invention, the concentrated erythrocytes solution is frozen in a dry ice-methanol solution, and the frozen solution is thawed out in a warm water bath at 30° to haemolyze the concentrated erythrocytes. By using the freezing and thawing method, the erythrocytes can be easily haemolyzed under sterile conditions without diluting the haemoglobins.

Finally, the haemoglobins are separated and purified, preferably without dilution of haemoglobin solution. Even if the concentrated erythrocytes are haemolyzed, the membrane constituents of the erythrocytes (stroma), low molecular-weight materials and the like are present together with the haemoglobins; these impurities should be removed. The method of removal is not particularly critical. For example, a centrifugal separation method can be used. Care must however then be taken to prevent the dilution of the haemoglobins.

As described above, when the process of the present invention is employed, the erythrocytes which can be easily concentrated can be concentrated as much as possible and then haemolyzed. Accordingly, the haemoglobins are efficiently obtained.

Merits of the present invention are as follows. Since the purified haemoglobins are produced by a process which comprises concentrating erythrocytes, haemolyzing the erythrocyte concentrate and removing membrane constituents (stroma) from the haemolyzed concentrate, as described above, it is possible to obtain efficiently a haemoglobin concentrate having a concentration of three or more times the concentration of haemoglobins which is obtained by conventional methods which comprise haemoglobins and concentrating the separated haemoglobins.

The following Examples illustrate the present invention more specifically.

Example 1

400 ml of human blood (preserved blood), which had expired its term of validity, was placed in four centrifuge tubes of 100 ml and centrifuged at 4°C at 3,000 rpm for 5 minutes. For separating erythrocytes and blood plasma, the supernatant of plasma ingredients was removed, a physiological sodium chloride solution having the same volume as that of the supernatant was added to the crude erythrocyte, the solution obtained was centrifuged under the same conditions as described above, and the supernatant obtained was removed. The operation was repeated twice and the purified erythrocyte concentrate was taken out of the apparatus. Then, the concentrate was placed in a 300 ml flask having an eggplant form. The flask was slowly rotated in a dry ice-methanol solution to freeze the erythrocytes and then placed in a hot water bath at 30°C to dissolve the frozen erythrocytes. The operation was repeated twice so as to completely haemolyze the erythrocytes. The haemolyzed solution was placed in a 40 ml centrifuge at 4°C at 25,000 rpm for 30 minutes. Stromamata of membrane constituents were removed as precipitates. The operation was repeated twice. Then, the haemoglobin solution obtained, which was not diluted, was passed through membrane filters having hole diameters of 5 μm, 3 μm, 2 μm, 1.2 μm, 0.8 μm, 0.45 μm and 0.2 μm, three times to each system, and the haemoglobins were thus purified.

The concentration of the haemoglobin concentrate obtained was 26.0% by weight and the Met-haemoglobin content was 8.0% and the quantity of phospholipid was 40 mg/dl.

Example 2

Using the same operation as used in Example 1, the erythrocyte concentrate was haemolyzed and centrifuged, and the precipitated stromamata were removed. Filtration was achieved by circulating the haemoglobin solution obtained through a hollow fibre module having a hole diameter of 0.2 μm. Since the haemoglobins easily passed through the module at 4°C and the stroma residue remained in the circulating liquid, the purification of the haemoglobins was finished in a short time.

The concentration of the haemoglobin concentrate obtained was 24.5% by weight, the Met-haemoglobin content was 4.0% and the quantity of phospholipid was 10 mg/dl.

Example 3

Using the same operation as used in Example 1, the erythrocyte concentrate was haemolyzed. Filtration was immediately achieved by circulating the haemoglobin solution obtained through a hollow fibre module having a hole diameter of 0.2 μm at 4°C and the stromamata were removed.

The concentration of the obtained haemoglobin concentrate was 23.0% by weight, the Met-haemoglobin content was 3.2% and the quantity of phospholipid was 22 mg/dl.

Example 4

Using the same operation as used in Example 1, the erythrocytes were washed by centrifugation. To the erythrocytes, a hypertonic solution in which the erythrocytes did not haemolyze was added. Water was released from the erythrocytes and the erythrocytes were contracted. The erythrocyte solution was centrifuged and the concentrate of the contracted erythrocytes was separated. Then, using the same operation as used in Example 1, the concentrate was haemolyzed and the haemoglobin solution obtained was purified.

In this case, the haemoglobins in the erythrocytes were concentrated still more. The concentration of the haemoglobin concentrate obtained was higher 33.0% by weight, the Met-haemoglobin content was 9.2% and the quantity of phospholipid was 45 mg/dl.

Example 5

Using the same operation as used in Example 1, the contracted erythrocytes were concentrated and the erythrocyte concentrate was haemolyzed. Removal of stromamata was immediately achieved by circulating the haemoglobin solution obtained through a hollow fibre module having a hole diameter of 0.2 μm at 4°C.

The concentration of the haemoglobin concentrate obtained was 31.0% by weight, the Met-haemoglobin content was 4.5% and the quantity of phospholipid was 11 mg/dl.

Example 6

Using the same operation as used in Example 1, the erythrocytes were concentrated. The haemalysis of the erythrocyte concentrate was achieved by ultrasonic exposure (Bath type) under a nitrogen atmosphere. Stromamata were immediately removed by circulating the obtained haemoglobin solution through a hollow fibre module having a hole diameter of 0.2 μm at 4°C.

The concentration of the haemoglobin concentrate obtained was 25.0% by weight, the Methaemoglobin content was 10.2% and the quantity of phospholipid was 60 mg/dl.

Example 7

Using the same operation as used in Example 1, the erythrocytes were concentrated. The haemalysis of the erythrocyte concentrate was achieved by an electric perforation method in which erythrocyte membranes were perforated by the exposure of high-voltage pulses. After the haemoglobin solution obtained had been centrifuged and stromamata removed, the solution was filtered by circulating through a hollow fibre module having a hole diameter of 0.2 μm. Since the haemoglobins easily passed through the module at 4°C and the stroma residue remained in the circulating liquid, the purification of the haemoglobins was finished in a short time.

The concentration of the haemoglobin concentrate obtained was 19.6% by weight, the Methaemoglobin content was 3.2% and the quantity of phospholipid was 7 mg/dl.

**Claims**

1. A process for producing haemoglobin concentrate, characterised in that the process comprises concentrating erythrocytes, haemolyzing the concentrate of the erythrocytes, and removing impurities, for example membrane constituents (stroma), from the haemolyzed concentrate to obtain the separated and purified haemoglobin concentrate.

2. A process according to claim 1, wherein the erythrocytes are contracted red blood cells.

3. A process according to either preceding claim, wherein the erythrocytes are concentrated by centrifugation.

4. A process according to claim 3, wherein the erythrocytes are washed and a hypertonic solution in which the erythrocytes do not haemolyze is added to the washed erythrocytes, prior to centrifugation.

5. A process according to any preceding claim, wherein a freezing and thawing method, an ultrasonic exposure method or an electric perforation method is used to haemolyze the concentrate of the erythrocytes.

6. A process according to any preceding claim, wherein the haemolyzed concentrate is purified and separated by centrifugation.